(19) Europäisches Patentamt
European Patent Office
Office européen des brevets

(11) Veröffentlichungsnummer: **0 015 514**
**B1**

(12) # EUROPÄISCHE PATENTSCHRIFT

(45) Veröffentlichungstag der Patentschrift: **26.06.85**

(21) Anmeldenummer: **80101006.7**

(22) Anmeldetag: **29.02.80**

(51) Int. Cl.$^4$: **C 07 F 9/50,** C 07 F 9/53, B 01 J 31/00, C 07 C 5/00, C 07 C 45/00

(54) **Optisch aktive tertiäre Phosphine, ihre Herstellung und ihre Verwendung für asymmetrische Synthesen.**

(30) Priorität: **03.03.79 DE 2908358**

(43) Veröffentlichungstag der Anmeldung: **17.09.80 Patentblatt 80/19**

(45) Bekanntmachung des Hinweises auf die Patenterteilung: **26.06.85 Patentblatt 85/26**

(84) Benannte Vertragsstaaten: **BE CH DE FR GB IT NL**

(56) Entgegenhaltungen:
**DE-A-2 161 200**

**TETRAHEDRON LETTERS, vol. 46 (1966) Pergamon Press, GB L. HORNER et al. "Optisch Aktive 1,2-Äthylen-Bis-Phosphoniumsalze und 1,2-Äthylen-Bis-Phosphine", Seiten 5783-5787**

**ANGEWANDTE CHEMIE (Int. Ed.), 18(8), 1979, Verlag Chemie GmbH, Weinheim, DE H. BRUNNER et al. "Asymmetric Hydrogenation of (Z)-alpha-(Acetyl-amino)-cinnamic Acid by a Rh/norphos Catalyst". Seiten 620-1.**

(73) Patentinhaber: **BASF Aktiengesellschaft
Carl-Bosch-Strasse 38
D-6700 Ludwigshafen (DE)**

(72) Erfinder: **Brunner, Henri, Dr.
Eichendorffstrasse 14
D-8411 Lappersdorf (DE)**
Erfinder: **Pieronczyk, Willigis
Neupruell 13A
D-8400 Regensburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 015 514 B1**

**Beschreibung**

Die vorleigende Erfindung betrifft optisch aktive tertiäre Phosphine, Verfahren zu ihrer Herstellung und ihre Verwendung in Form ihrer Komplexverbindungen mit Metallen der VIII. Gruppe des Periodensystems als Katalysatoren für asymmetrische Synthesen wie die asymmetrische Hydrierung und Hydroformylierung olefinisch ungesättigter Verbindungen.

Es ist allgemein bekannt, daß Komplexverbindungen aus einem Metall der VIII. Gruppe des Periodensystems als Zentralatom (im folgenden als Z bezeichnet) und einem oder mehreren Molekülen eines tertiären Phosphins als Liganden hervorragend als Katalysatoren für die Hydrierung und Hydroformylierung olefinisch ungesättigter Verbindungen geeignet sind.

Diese grundsätzlich auf beliebige olefinisch ungesättigte Verbindungen anwendbaren Reaktionen, lassen sich an einem einfachen Beispiel, das im Hinblick auf die nachstehenden Betrachtungen gewählt wurde, wie folgt veranschaulichen:

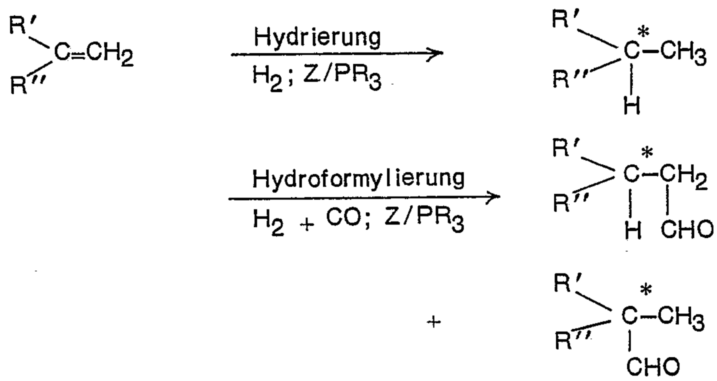


Hierin bedeuten R′ und R″ Wasserstoff oder organische Reste und R die organischen Reste des tertiären Phosphins.

Wie man erkennt, können hierbei die asymmetrischen C*-Atome entstehen, sofern sich $R^1$ und $R^2$ untereinander und von den übrigen beiden Resten an diesem C*-Atom unterscheiden. Verwendet man Katalysatoren ohne Phosphinliganden oder solche mit Phosphinen wie Triphenylphosphin oder Tributylphosphin als Liganden, so erhält man in diesen Fällen racemische Gemische als Verfahrensprodukte.

Bei Synthesen auf dem Gebiet physiologisch wirksamer Verbindungen, z.B. Arzneimitteln, Pflanzenschutzmitteln, Futtermitteln oder Duftstoffen, kommt es meistens jedoch auf die Konfiguration dieser Stoffe und damit auch auf die Konfiguration von deren Vorprodukten an. Zwar kann man die Racemate nach den bekannten Methoden über diastereomere Verbindungen trennen, jedoch sind diese Methoden häufig äußerst diffizil und dementsprechend kostspielig, und zwar nicht zuletzt auch deswegen, weil man für die Racematspaltung äquimolare Mengen des optisch aktiven Spaltreagenzes benötigt.

Aus den zusammenfassenden Arbeiten in Synthesis, Band 5 (1978), S. 329 und Pure Appl. Chem., Band 43 (1975) S. 401 ist es nun weiterhin bekannt, daß die Hydrierung und die Hydroformylierung von prochiralen Olefinen, d.h. von solchen Olefinen, die Verfahrensprodukte mit asymmetrischen C*-Atomen liefern, weitgehend stereospezifisch verlaufen, wenn man hierzu Katalysatoren des allgemeinen Typs $Z/PR_3$ verwendet, in denen das tertiäre Phosphin eine reine optisch aktive Verbindung ist. Sind die drei Reste R verschieden, so ist das P-Atom selber ein chirales Zentrum, jedoch kann sich das chirale Zentrum des optisch aktiven Phosphin-Liganden auch an anderer Stelle des Moleküls befinden. Die Verwendung derartiger Katalysatoren bietet den Vorteil des Potenzierungseffektes, d.h. mit einer geringen Menge eines optisch aktiven Katalysators erhält man große Mengen eines Hydrierungs- bzw. Hydroformylierungsproduktes mit überwiegendem Anteil eines der beiden optischen Isomeren.

So attraktiv dieses Verfahrensprinzip ist, so bietet seine technische Realiserung doch erhebliche Schwierigkeiten, und zwar vor allem deshalb, weil die hierfür geeigneten tertiären Phosphine bereits in der Racematform schwer zugänglich sind und weil die Trennung der Racemate in ihre optisch aktiven Antipoden nur mit großem experimentellem Aufwang gelingt.

Unter den Aufgaben der vorliegenden Erfindung steht deshalb diejenige eines allgemein anwendbaren Verfahrens zur Herstellung optisch reiner tertiärer Phosphine im Vordergrund. Weiterhin war es Aufgabe der Erfindung, die Technik um neue derartige Phosphine zu bereichern, die leichter zugänglich sind als die bisher bekannten und die als Liganden in der für die Hydrierung und Hydroformylierung prochiraler Olefine verwendeten Komplexkatalysatoren eine erhöhte Stereospezifität hinsichtlich eines der beiden optischen Isomeren bewirken.

Es wurde gefunden, daß man Racemate optisch aktiver tertiärer Phosphine trennen kann, wenn man

— die Phosphine zunächst in an sich bekannter Weise in die entsprechenden Phosphinoxide überführt,

— die so erhaltenen racemischen Gemische der Phosphinoxide nach den Prinzipien der Enantiomerentrennung über stereomere Verbindungen in organischer Lösung mit einem optisch reinen

Isomeren einer an den alkoholischen Hydroxylgruppen mono- oder bisacylierten Weinsäure umsetzt,
— die in dem Lösungsmittel schwerer lösliche diastereomere Verbindung abtrennt,
— das Weinsäurederivat mittels einer Base von der erhaltenen reinen diastereomeren. Verbindung wieder abspaltet und
— die so erhaltenen optisch reinen oder bereits weitgehend optisch reinen Phosphinoxide in an sich bekannter Weise wieder zu den Phosphinen reduziert.

In einer alternativen Ausführungsform zu diesem Verfahren kann man auch unmittelbar von (D,L)-Phosphinoxiden ausgehen, sofern diese auf anderem Wege als über die entsprechenden (D,L)-Phosphine zugänglich sind.

Dieses allgemein anwendbare Verfahren ist deshalb besonders bemerkenswert, weil die Phosphinoxide wider Erwarten mit den definitionsgemäßen Weinsäurederivaten diastereomere Umsetzungsprodukte liefern, wobei es sich höchstwahrscheinlich um Wasserstoff-Brücken-Addukte beider Verbindungen handelt.

Das Verfahren läßt sich durch folgendes Schema veranschaulichen:

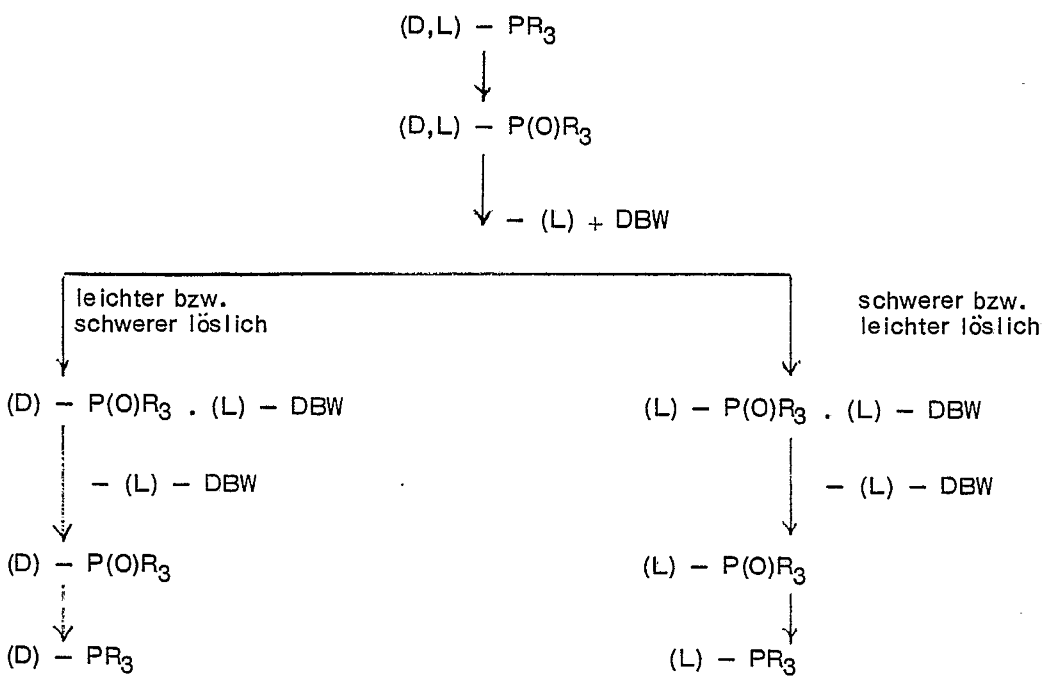


R = organische Reste
DBW = Dibenzoylweinsäure

Das erfindungsgemäße Verfahren kann z.B. auf die Racemattrennung folgender bekannter chiraler Phosphine angewendet werden:

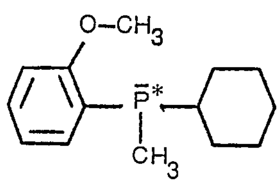
o-Methoxyphenyl-methyl-cyclohexylphosphin

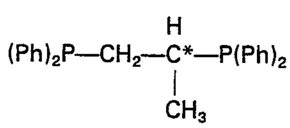
1,2-Bis(diphenylphosphino)propan

*Chiralitätszentrum
Ph = Phenyl

Besonders hervorragend stereospezifisch-katalytische Eigenschaften haben die reinen optischen Isomeren des 2,3-Bis(diphenylphosphino)-bicyclo[2,2,1]hept-5-en (I)

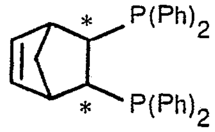 I

Sowohl das Racemat von I als auch die (D)- und die (L)-Form sind neue Verbindungen, die nach dem erfindungsgemäßen Verfahren aus bekannten Vorprodukten erhältlich sind. Hierbei geht man von dem racemischen Bis-phosphinoxid (II)

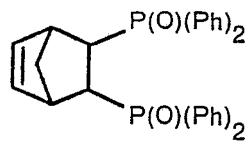 II

aus, welches man in äthanolischer Lösung mit der äquimolaren Menge des Weinsäurederivates, und zwar vorzugsweise von (−)-(L)-Dibenzoylweinsäuremonohydrat ((−)-(L)-DBW) versetzt. Das hierbei als weißer Niederschlag anfallende diastereomere Wasserstoffbrücken-Addukt (−)-II/(−)-(L)-DBW ist in Äthanol sowie in den meisten organischen Lösungsmitteln schwerer löslich als das Diastereomere (+)-II/(−)-(L)-DBW. Als weitere Lösungsmittel sind neben Äthanol z.B. Methanol, Propanol, iso-Propanol, Butanol, Chloroform, Methylenchlorid, Benzol und Toluol geeignet. Vorzugsweise nimmt man die Umsetzung bei Raumtemperatur vor, jedoch kann man allgemein Temperaturen zwischen etwa 0 und 50°C anwenden. Fällt das Diastereomere nicht von allein aus, so kann man die Kristallisation mittels einiger Impfkristalle und gegebenenfalls auch mittels Temperatursenkung beschleunigen. Die Menge des Lösungsmittels wird hierbei zweckmäßigerweise so bemessen, daß diese zur vollständigen Lösung von II gerade ausreicht. Diese Menge, die unschwer zu ermitteln ist, entspricht im Falle der oben gennanten Lösungsmittel etwa 15 bis 30 gew.%igen Lösungen von II.

Kristallisat und Lösung werden sodann auf gleiche Weise auf die optischen Isomeren (+)-II und (−)-II aufgearbeitet, wobei man im Falle der Lösung das Lösungsmittel abzieht, d.h. man überführt das zunächst gelöste Diastereomere ebenfalls in die feste Form. In beiden Fällen löst oder suspendiert man das feste Diastereomere zweckmäßigerweise in einem Lösungsmittel, welches mit Wasser nicht mischbar ist, z.B. in Chloroform, Methylenchlorid, Benzol oder Toluol und versetzt die Lösung bzw. die Suspension sodann mit einer verdünnten wäßrigen Alkalilauge, z.B. mit KOH oder NaOH. Hierbei wird die DBW in Form ihres Alkalisalzes abgespalten, welches in die wäßrige Phase geht. Die DBW kann sodann wie üblich wieder zurückgewonnen werden, z.B. indem man diese Lösung über einen sauren Ionenaustauscher leitet. Hierbei erhält man eine wäßrige DBW-Lösung, aus der man die DBW durch Abziehen des Wassers oder durch Extraktion mit Äther wieder in reiner Form erhält.

Bei der Spaltung des Diastereomeren geht das Phosphinoxid II in die organische Phase, aus der es wie üblich isoliert werden kann. Die optische Reinheit beträgt nach diesem Trennungsgang etwa 76% und kann bereits durch einmalige Wiederholung des Verfahrens auf praktisch 100% erhöht werden, d.h. mit weiteren Reinigungsstufen nimmt der Drehwinkel nicht mehr zu.

Sofern das Lösungsmittel inert ist, kann man die II-Lösung unmittelbar der Reduktionsreaktion unterwerfen. Als Reduktionsmittel eignet sich hierfür, wie aus J.Am. Chem. Soc., Band 99 (1977), S. 5950 und der dort zitierten Vorliteratur bekannt ist, besonders Trichlorsiliciumwasserstoff ($HSiCl_3$).

Man führt die Reduktion zweckmäßigerweise mit 4 bis 10 mol $HSiCl_3$ pro Mol II in organischer Lösung, z.B. in Benzol bei 60 bis 100°C und unter einem Druck von 1 bis 10 bar aus, wonach man das Gemisch bei 5 bis 30°C mit einer wäßrigen Alkalilauge, z.B. NaOH versetzt. Nach Abtrennung der wäßrigen Phase destilliert man das Lösungsmittel von der organischen Phase, wobei das Phosphin I zurückbleibt. Zur weiteren Reinigung kann man es in Aceton umkristallisieren.

Das bekannte racemische Phosphinoxid II ist auf folgendem Wege erhältlich: Nach der Vorschrift in J.Am. Chem. Soc., 86 (1964), S. 2299 setzt man trans-1,2-Dichloräthylen mit Kaliumdiphenylphosphin zu dem prochiralen trans-1,2-Diphenylphosphinoäthylen

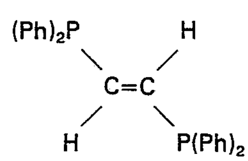

um, oxidiert diese Verbindung mit Wasserstoffperoxid zum entsprechenden Phosphinoxid und setzt dieses nach dem Verfahren von Izv. Akad. Nauk SSSR Ser. Khim (engl.), 10, (1974), S. 2210 in einer Diels-Alder-Reaktion mit Cyclopentadien zum II-Racemat um.

Die am Beispiel der Herstellung von I und dessen reinen optischen Isomeren angegebenen Verfahrensvorschriften gelten ebenso für die Verwendung der übrigen Weinsäurederivate, die ganz

allgemein die Mono- oder Bisacylate der alkoholischen Hydroxylgruppen der Weinsäure sein können. Die Acylreste können sich dabei von aliphatischen, aromatischen, cycloaliphatischen und araliphatischen organischen Säuren mit vorzugsweise 1 bis 12 C-Atomen ableiten, die ihrerseits inerte Substituenten tragen können. Unter diesen Spaltungsreagenzien hat sich die Dibenzoylweinsäure als besonders geeignet erwiesen.

In einer Abänderung des Trennungsverfahrens kann man häufig auch von der halben molaren Menge der (L)-DBW, bezogen auf das racemische II, ausgehen. In diesem Falle erhält man nur das schwerer lösliche Diastereomere (−)-II/(−)-(L)-DBW, wogegen das antipodische (+)-II in Lösung verbleibt.

Die genannten Vorschriften sind, gegebenenfalls nach einigen Orientierungsversuchen, auf die Herstellung beliebiger optisch reiner Phosphine, ausgehend von den racemischen Phosphinen oder Phosphinoxiden anwendbar.

Wie allgemein bekannt ist, bilden tertiäre Phosphine mit den Metallen Z der VIII. Gruppe des Periodensystems Komplexverbindungen des Typs

$$Z^r/(PR_3)_n/L_{m-n}/A_r$$

wobei L sonstige Liganden sein können, n einen Wert von 1 bis m hat und m die Gesamtwertigkeit in Bezug auf die Liganden am Zentralatom Z ist, r die Wertigkeit des Zentralatoms ist und A für ein Anion steht. Als Metalle Z kommen für die Zwecke der Hydrierung vornehmlich Ruthenium, Iridium, Palladium, Platin und Cobalt sowie besonders Rhodium in Betracht und für die Hydroformylierung Rhodium und Cobalt. Sonstige Liganden sind z.B. Cyclooctadien, Acetylacetonat und Kohlenmonoxid. Z selber kann in nullwertiger (r = 0) Form vorliegen oder, als salzartige Komplexverbindung, in höherwertiger Form.

Im letzteren Falle kommen als weitere Komponenten in der Komplexverbindung noch die Anionen A wie $Cl^-$ oder $Br^-$ in der der Wertigkeit entsprechenden Anzahl hinzu.

Zahlreiche Komplexverbindungen dieser Art sind bekannt, desgleichen deren Herstellung. Für den Fall, daß $PR_3$ ein optisch aktiver Ligand ist, gelten hier naturegemäß keinerlei Besonderheiten. Dies gilt auch für die Methodik der Hydrierung und der Hydroformylierung, so daß nähere Angaben sich hierzu erübrigen. Häufig bilden sich derartige oder ähnliche Komplexe unter den Reaktionsbedingungen in situ, so daß es genügt, ein Z-Salz oder einen phosphinfreien Z-Komplex und das Phosphin getrennt in den Reaktionsansatz zu geben. Wie bei sonstigen Hydrierungen und Hydroformylierungen kann es sich hierbei empfehlen, freies Phosphin in einem etwa bis zu 10-molaren Überschuß über die Komplexverbindung mitzuverwenden.

Gemäß der Aufgabenstellung der Erfindung verwendet man die stereospezifischen Katalysatoren naturgemäß nur zur Hydrierung bzw. Hydroformylierung prochiraler olefinisch ungesättigter Verbindungen, wie es eingangs am Beispiel des Olefins $R'R''C=CH_2$ erläutert wurde. Selbstverständlich gilt dies auch für Olefine des Typs $R'R''C=CR'''R''''$, wobei u.U. auch zwei chirale Zentren entstehen können. Ferner ist zu berücksichtigen, daß man bei der Hydroformylierung in aller Regel Isomerengemische erhält, und zwar je nachdem, an welchem C-Atom die Formylgruppe in das Molekül eintritt. In allen Fällen erhält man indes überwiegende Mengen eines der optischen Isomeren, wobei je nach der Racemisierungsneigung der Verfahrensprodukte optische Reinheiten bis zu 95% erreichtwerden können. Für praktische Zwecke können jedoch auch optische Reinheiten ab etwa 10% wirtschaftlich von Bedeutung sein.

## Beispiel 1

trans-(−)-2,3-Bis(diphenylphosphinoxido)-bicyclo-[2,2,1]-hept-5-en; (−)-II

Eine Lösung aus 25,5 g des racemischen II und 105 ml 99%igem Äthanol wurde bei Raumtemperatur mit einer Lösung aus 19,5 g (−)-(L)-Dibenzoylweinsäuremonohydrat (−)-(L)-DBW in 20 ml Äthanol versetzt. Das Molverhältnis von II zu DBW betrug 1:1.

Nach etwa 2 Minuten begann das schwerer lösliche Diastereomere (−)-II/(−)-(L)-DBW als weißer Niederschlag auszufallen. Die Diastereomerenbildung war nach 1 Stunde beendet. Das leichter lösliche Diastereomere (+)-II/(−)-(L)-DBW verblieb in Lösung.

Der trockene Niederschlag (Ausbeute rd. 43%) wurde sodann in 100 ml Chloroform aufgenommen und mit einer Lösung aus 4 g KOH und 160 ml Wasser intensiv verrührt.

Die wäßrige Phase wurde abgetrennt und einmal mit 30 ml Chloroform gewaschen. Beide Chloroformphasen werden vereinigt, getrocknet und destillativ bei Raumtemperatur vom Chloroform befreit.

Die Rohausbeute an (−)-II betrug 43% (= 11 g). Drehwinkel des Rohproduktes:

$[\alpha]^{20}_{578}$ (c 1; Chloroform) = −47°

Die einmalige Wiederholung dieses Trennverfahrens lieferte das reine (−)-II in 35%iger Ausbeute und praktisch völliger Reinheit, da bei weiteren Wiederholungen keine weitere Drehwertverbesserung mehr zu beobachten war.

(−)-II, Drehwinkel: $[\alpha]^{20}_{578}$ (c 1; Chloroform) = −62°

Beispiel 2

trans-(+)-2,3-Bis(diphenylphosphinoxido)-bicyclo-[2,2,1]-hept-5-en; (+)-II

Die nach Beispiel 1 erhaltene äthanolische Lösung des leichter löslichen Diastereomeren (+)-II/(−)-(L)-DBW wurde eingeengt, wonach der Rückstand mit 100 ml wasserfreiem Aceton verrührt wurde. Hierbei ging das noch vorhandene schwerer lösliche Diastereomere nicht in Lösung. Dieser Rückstand wurde abgetrennt (4,5 g), wonach das in Lösung verbliebene Diastereomere analog Beispiel 1 auf das (+)-II aufgearbeitet wurde.

Drehwinkel der reinen (+)-(II):

$[\alpha]^{20}_{578}$ (c 1; Chloroform) = +58°

Beispiel 3

trans-(+)-2,3-Bis(diphenylphosphino)-bicyclo-[2,2,1]-hept-5-en; (+)-I

Eine Mischung aus 9 g (+)-II, 15 g Trichlorsiliciumwasserstoff und 200 ml Benzol wurde 15 Stunden lang unter Eigendruck (rd. 3 bar) auf 75°C erhitzt. Nach Abdestillieren der überschüssigen Si-Verbindung wurde der Rückstand in 100 ml Benzol aufgenommen und bei 6°C tropfenweise mit soviel 25 gew.%iger Natronlauge versetzt, bis sich der zunächst anfallende Niederschlag (wahrscheinlich von SiOH-Verbindungen) wieder gelöst hatte. Die benzolische Phase wurde mit Wasser gewaschen und getrocknet, wonach das Benzol abdestilliert wurde. Der Rückstand wurde aus heißem Aceton umkristallisiert. Die Ausbeute an (+)-I betrug 75%. Schmelzplunkt 129—130°C.

Beispiel 4

trans-(−)-2,3-Bis(diphenylphosphino)-bicyclo[2,2,1]-hept-5-en; (−)-I

Analog Beispiel 3 wurde das (−)-II zu dem optisch reinen (−)-I reduziert; Smp. 129—130°C.

Drehwinkel $[\alpha]^{20}_{578}$ (c 1; Chloroform) = −43,5°

Beispiel 5

Herstellung von (D-)-N-Acetylphenylalanin

Zur Bereitung eines stereospezifischen Hydrierungskatalysators wurden 7 mg (1,4 . $10^{-5}$ mol) der Komplexverbindung Bis-(cycloocta-1,5-dien)-μ,μ'-dichlorodirhodium

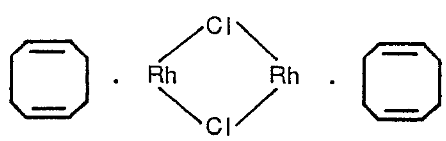

und 14,5 mg (3,1 . $10^{-5}$ mol) (+)-I in 5 ml Methanol gelöst und 30 min gerührt. Hierbei bildete sich eine gelborange Lösung einer Komplexverbindung aus dem eingesetzten Rh-Komplex und dem Phosphin.

Diese Katalysatorlösung wurde mit einer Lösung aus 500 mg (2,4 . $10^{-3}$ mol) der prochiralen Verbindung (Z)-α[N-Acetamino]-zimtsäure

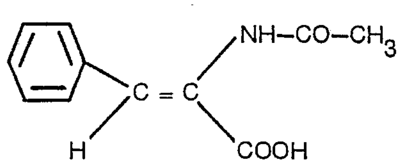

vermischt und anschließend bei Raumtemperatur unter einem Wasserstoffdruck von 1 bis 1,2 bar wie üblich hydriert. Die Wasserstoffaufnahme betrug etwa 50 ml.

Die Lösung wurde sodann unter vermindertem Druck zur Trockene eingedampft, wonach der Rückstand in 5 ml 0,5-normaler wäßriger Natronlauge aufgenommen wurde. Hierbei zersetzte sich der Katalysator, dessen Komponenten als fester Rückstand anfielen. Dieser Rückstand wurde dreimal mit je 3 ml Wasser gewaschen. Die alkalische Lösung und das Waschwasser wurden vereinigt, mit 2,6 ml 1-n-HCl leicht angesäuert und danach mit Äther extrahiert. Die übliche Aufarbeitung der ätherischen Extraktphase lieferte das N-Acetylphenylalanin in praktisch quantitativer Ausbeute, wobei die optische Ausbeute bezüglich des (D-)-Isomeren rund 95% betrug, wie sich aus dem Literaturdrehwert (Tetrahedron Letters 52, 1977, S. 4639) ergab.

Beispiel 6

Herstellung von (L+)-N-Acetylphenylalanin

Diese Verbindung wurde auf die in Beispiel 5 angegebene Weise, jedoch mit der antipodischen Katalysatorkomponente (−)-I in gleicher Ausbeute und gleicher Reinheit hergestellt, wie das (D-)-N-Acetylphenylalanin.

Beispiel 7

Herstellung von (−)-1,2-Bis-diphenylphosphinoxido-propan; III

Eine Lösung von 15,9 g racemischem III in 75 ml 99%igem Äthanol wurde bei Raumtemperatur mit 12,5 g (−)-(L)-DBW versetzt. Das Molverhältnis der beiden Diastereomerenkomponenten betrug 1:1 Nach 20-stündigem Rühren wurden 4,2 g des schwerer löslichen Diastereomeren (−)-III/(−)-(L)-DBW abgetrennt, getrocknet und in einem Gemisch aus 10 ml 1n-NaOH und 40 ml Chloroform gelöst. Aus der organischen Phase wurden 2 g angespeichertes (−)-III isoliert.

Drehwert $[\alpha]^{20}_{578}$ (c 1; Chloroform) = −8°

Beispiel 8

Herstellung von (+)-2-Phenylpropanal

In einem Hochdruckautoklaven von 1 l Inhalt wurden 100 g Styrol in 600 ml Toluol als Lösungsmittel mit 50 g dimerem Rhodiumcarbonylchlorid und 250 mg (+)-II bei 80°C und 200 bar mit einem Gemisch aus 45 Vol.% CO und 55 Vol.% $H_2$ innerhalb von 12 Stunden hydroformyliert. Die übliche Aufarbeitung des Reaktionsgemisches lieferte das optisch inaktive 3-Phenylpropanal in rd. 9%iger Ausbeute und ein Gemisch der optischen Isomeren des 2-Phenylpropanal in rd. 78%iger Ausbeute. Ensprechend dem Drehwinkel $[\alpha]^{20}_{D}$ (c 1; Chloroform) = +48°C betrug die optische Reinheit des 2-Phenylpropanals 22%.

**Patentansprüche**

1. Verfahren zur Herstellung reiner optisch aktiver tertiärer Phosphine durch Spaltung der entsprechenden racemischen Verbindungen, dadurch gekennzeichnet, daß man

— die Phosphine zunächst in an sich bekannter Weise in die entsprechenden Phosphinoxide überführt,

— die so erhaltenen racemischen Gemische der Phosphinoxide nach den Prinzipien der Enantiomerentrennung über stereomere Verbindungen in organischer Lösung mit einem optisch reinen Isomeren einer an den alkoholischen Hydroxylgruppen mono- oder bisacylierten Weinsäure umsetzt,

— die in dem Lösungsmittel schwerer lösliche diastereomere Verbindung abtrennt,

— das Weinsäurederivat mittels einer Base von der erhaltenen reinen diastereomeren Verbindung wieder abspaltet und

— die so erhaltenen optisch reinen oder bereits weitgehend optisch reinen Phosphinoxide in an sich bekannter Weise wieder zu den Phosphinen reduziert.

2. Alternative Ausführungsform des Verfahrens gemäß Anspruch 1, dadurch gekennzeichnet, daß man von racemischen Phosphinoxiden ausgeht, die auf anderem Wege als über die entsprechenden (D,L)-Phosphine hergestellt worden sind.

3. Verfahren nach Anspruch 2, dadurch gekennzeichnet, daß man es auf die Spaltung von trans-(D,L)-2,3-Bis(diphenylphosphinoxido)-bicyclo[2,2,1]hept-5-en anwendet.

4. Verfahren nach den Ansprüchen 1 bis 3, dadurch gekennzeichnet, daß man als Spaltreagenz (D)-bzw. (L)-Dibenzoylweinsäure verwendet.

5. trans-(D)-2,3-Bis(diphenylphosphino)-bicyclo[2,2,1]-hept-5-en.

6. trans-(L)-2,3-Bis(diphenylphosphino)-bicyclo[2,2,1]-hept-5-en.

7. Verfahren zur stereospezifischen Hydrierung prochiraler olefinisch ungesättigter Verbindungen mittels Wasserstoff und Katalysatoren des Typs

$$Z^r/(PR_3)_n/L_{m-n}/A_r$$

wobei Z ein Metall der VIII. Gruppe des Periodensystems ist, $PR_3$ ein tertiäres Phosphin, L einen sonstigen Liganden und A ein Anion in der der Wertigkeit r des Zentralatomes entsprechenden Anzahl bedeuten, n einen Wert von 1-m hat und m die Gesamtwertigkeit in Bezug auf die Liganden am Zentralatom Z ist, in an sich bekannter Weise, dadurch gekennzeichnet, daß man als Phosphin $PR_3$ ein optisch reines Isomeres gemäß den Ansprüchen 5 bzw. 6 verwendet.

8. Verfahren zur stereospezifischen Hydroformylierung prochiraler olefinisch ungesättigter Verbindungen mit Wasserstoff und Kohlenmonoxid und Katalysatoren des Typs

$$Z^r/(PR_3)_n/L_{m-n}/A_r$$

wobei Z ein Metall der VIII. Gruppe des Periodensystems ist, $PR_3$ ein tertiäres Phosphin, L einen sonstigen Liganden und A ein Anion in der der Wertigkeit r des Zentralatomes entsprechenden Anzahl bedeuten, n einen Wert von 1-m hat und m die Gesamtwertigkeit in Bezug auf die Liganden am Zentralatom Z ist, in an sich bekannter Weise, dadurch gekennzeichnet, daß man als Phosphin $PR_3$ ein optisch reines Isomeres gemäß den Ansprüchen 5 bzw. 6 verwendet.

**Revendications**

1. Procédé de préparation de phosphines tertiaires optiquement actives pures par dédoublement des racémiques correspondants, caractérisé en ce que:

— on transforme d'abord les phosphines de manière connue en soi en les oxydes de phosphine correspondants,
— on fait ensuite réagir les mélanges racémiques d'oxydes de phosphine ainsi obtenus, selon des principes de la séparation des énantiomères par passage par des composés stéréomères, en solution organique avec un isomère optiquement pur d'un acide tartrique mono- ou bis-acylé sur les groupes hydroxyle alcooliques,
— on sépare le composé diastéréomère le moins soluble dans le solvant,
— on sépare le dérivé de l'acide tartrique à l'aide d'une base du composé diastéréo-isomère pur obtenu et
— on retransforme les oxydes de phosphine optiquement purs ou à peu près purs obtenus de manière connue en soi par réduction en phosphines.

2. Variante du procédé suivant la revendication 1, caractérisée en ce que l'on emploie des oxydes de phosphine racémiques préparés par une autre voie qu'au départ des (D,L)-phosphines.

3. Procédé suivant la révendication 2, caractérisé en ce qu'il est appliqué au dédoublement du (D,L)-2,3-bis-(diphénylphosphinoxydo)-bicyclo[2,2,1)hept-5-ène trans.

4. Procédé suivant les revendications 1 à 3, caractérisé en ce que l'on utilise comme réactif de dédoublement l'acide (D)- ou (L)-dibenzoyl-tartrique.

5. Le (D)-2,3-bis-(diphényl-phosphino)-bicyclo [2,2,1]-hept-5-ène trans.

6. Le (L)-2,3-bis-(diphényl-phosphino)-bicyclo [2,2,1]-hept-5-ène trans.

7. Procédé pour l'hydrogénation stéréo-spécifique de composés à insaturation oléfinique prochiraux par l'hydrogène en présence de catalyseurs du type

$$Z^r/(PR_3)_n/L_{m-n}/A_r$$

où Z désigne un métal du groupe VIII du Système périodique, $PR_3$ représente une phosphine tertiaire, L désigne un autre ligand et A un anion, dont le nombre correspond à la valence r de l'atome central, n = 1-m et m correspond à la valence totale par rapport aux ligands sur l'atome central Z, de manière connue en soi, caractérisé en ce que l'on emploie comme phosphine $PR_3$ l'un des isomères optiquement purs selon la revendication 5 ou la revendication 6.

8. Procédé d'hydroformylation stéréo-spécifique de composés à insaturation oléfinique prochiraux par l'hydrogène et l'oxyde de carbone en présence de catalyseurs du type

$$Z^r/(PR_3)_n/L_{m-n}/A_r$$

où Z désigne un métal du groupe VIII du Système périodique, $PR_3$ représente une phosphine tertiaire, L désigne un autre ligand et A un anion, dont le nombre correspond à la valence r de l'atome central, n = 1-m et m correspond à la valence totale par rapport aux ligands sur l'atome central Z, de manière connue en soi, caractérisé en ce que l'on emploie comme phosphine $PR_3$ l'un des isomères optiquement purs selon la revendication 5 ou la revendication 6.

## Claims

1. A process for the preparation of pure optically active tertiary phosphines by resolving the corresponding racemic compounds, wherein
— the phosphines are first converted into the corresponding phosphine oxides in a conventional manner,
— the resulting racemic mixtures of the phosphine oxides are reacted in organic solution, in accordance with the principles of enantiomer separation via stereomeric compounds, with an optically pure isomer of a tartaric acid which is mono-acylated or bis-acylated at the alcoholic hydroxyls,
— the diastereomeric compound which is less soluble in the solvent is separated off,
— the tartaric acid derivative is split off from the resulting pure diastereomeric compound by means of a base, and
— the resulting optically pure or substantially optically pure phosphine oxides are reduced back to the phosphines in a conventional manner.

2. An alternative embodiment of the process as claimed in claim 1, wherein racemic phosphine oxides which have been prepared by a route other than via the corresponding (D,L)-phosphines are used as starting materials.

3. A process as claimed in claim 2, wherein trans-(D,L)-2,3-bis-(diphenylphosphine-oxido)-bicyclo[2,2,1]-hept-5-ene is resolved.

4. A process as claimed in claims 1 to 3, wherein (D)- or (L)-dibenzoyltartaric acid is used as the resolving agent.

5. trans-(D)-2,3-Bis-(diphenylphosphino)-bicyclo[2,2,1]-hept-5-ene.

6. trans-(L)-2,3-Bis-(diphenylphosphino)-bicyclo[2,2,1]-hept-5-ene.

7. A process for the stereospecific hydrogenation, in a conventional manner, of prochiral olefinically unsaturated compounds by means of hydrogen and a catalyst of the type

$$Z^r/(PR_3)_n/L_{m-n}/A_r$$

where Z is a metal of group VIII of the periodic table, $PR_3$ is a tertiary phosphine, L is another ligand and A is an anion present in a number corresponding to the valency r of the central atom, n is from 1 to m and m is the total valency of the central atom Z in respect of the ligands, wherein an optically pure isomer as claimed in claim 5 or 6 is used as the phosphine $PR_3$.

8. A process for the stereospecific hydroformylation, in a conventional manner, of prochiral olefinically unsaturated compounds with hydrogen and carbon monoxide and a catalyst of the type

$$Z^r/(PR_3)_n/L_{m-n}/A_r$$

where Z is a metal of group VIII of the periodic table, $PR_3$ is a tertiary phosphine, L is another ligand and A is an anion present in a number corresponding to the valency r of the central atom, n is from 1 to m and m is the total valency of the central atom Z in respect of the ligands, wherein an optically pure isomer as claimed in claim 5 or 6 is used as the phosphine $PR_3$.